(19) Europäisches Patentamt
European Patent Office
Office européen des brevets

(11) EP 0 885 897 B1

(12) **EUROPÄISCHE PATENTSCHRIFT**

(45) Veröffentlichungstag und Bekanntmachung des Hinweises auf die Patenterteilung:
**21.08.2002 Patentblatt 2002/34**

(21) Anmeldenummer: **98110000.1**

(22) Anmeldetag: **02.06.1998**

(51) Int Cl.[7]: **C07F 9/655**, C07F 15/00, B01J 31/24, C07B 53/00, C07C 45/50 // C07M7:00

# (54) Optisch aktive Diphosphinliganden

Optically active diphosphine ligands

Ligands diphosphines optiquement actifs

(84) Benannte Vertragsstaaten:
**CH DE ES FR GB IT LI**

(30) Priorität: **18.06.1997 DE 19725643**

(43) Veröffentlichungstag der Anmeldung:
**23.12.1998 Patentblatt 1998/52**

(73) Patentinhaber: **BASF AKTIENGESELLSCHAFT 67056 Ludwigshafen (DE)**

(72) Erfinder:
- **Helmchen, Günther, Prof. Dr. 69115 Heidelberg (DE)**
- **Mürmann, Christoph, Dr. 67105 Schifferstadt (DE)**

(56) Entgegenhaltungen:
**EP-A- 0 000 403** **EP-A- 0 184 872**
**WO-A-97/13763**

- **JANSEN J F G A ET AL: "Michael addition of lithiodiphenylphosphine to menthyloxy-2[5H]-furanone: enantioselective synthesis of S,S-CHIRAPHOS" TETRAHEDRON: ASYMMETRY (TASYE3,09574166);90; VOL.1 (10); PP.719-20, XP002078418 Univ. Groningen;Dep. Org. Chem.; Groningen; 9747 AG; Neth. (NL)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft optisch aktive Diphosphine, ein Verfahren zur Herstellung optisch aktiver Diphosphine und deren Übergangsmetallkomplexe sowie die Anwendung der Metallkomplexe für die enantioselektive Synthese.

Für die Synthese optisch aktiver Verbindungen spielt die enantioselektive Hydrierung und Isomerisierung mit Rhodium und Rutheniumkomplexen eine große Rolle [z.B. Tani et al., J. Am. Chem. Soc. 106, 5211, (1984); R. Noyori, Acc. Chem. Res. 23, 345 (1990)]. Allerdings sind die dabei zur Anwendung kommenden Katalysatoren, die meist aus einem optisch aktiven Diphosphinliganden und einer Rhodium- oder Rutheniumverbindung hergestellt werden, sehr teuer und nur durch aufwendige Herstellverfahren zugänglich.

Die bekannten Herstellmethoden für optisch aktive Phosphine und Diphosphine sind durchweg umständlich und schließen meist eine technisch aufwendige und teure Racematspaltung ein (z.B. EP-A-0 614 901; EP-A-0 271 311; H.B. Kagan, "Chiral Ligands for Asymmetric Catalysis" in Asymmetric Synthesis, Vol. 5 (1985), Seite 13-23, EP-A-0 151 282; EP-A-0 185 882; R. Noyori, Acc. Chem. Res. 23, 345 (1990); EP-A-0 269 395; M.J. Burk, Tetrahedron, Asymmetry, S. 569-592 (1991); J. Am. Chem. Soc. 113, 8518-19 (1991), ibid. 115, 10125-138 (1993), ibid. 117, 9375-76 (1995), ibid 118, 5142 (1996)). Diese Nachteile machen eine industrielle Nutzung solcher Katalysatoren bislang schwierig und unwirtschaftlich.

Es bestand daher die Aufgabe, optisch aktive Diphosphine bereitzustellen, die sich als Liganden für Übergangsmetallkomplexkatalysatoren eignen, bei deren Herstellung die oben angegebenen Nachteile vermieden werden.

Diese Aufgabe wurde gelöst durch die Bereitstellung optisch aktiver Diphosphine der Formel I,

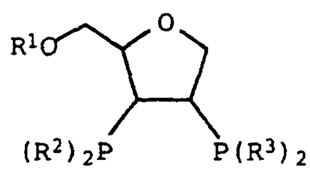

I

in der die Substituenten folgende Bedeutung haben:

$R^1$ Wasserstoff;
$C_1$-$C_{10}$-Acyl, $C_1$-$C_{10}$-Alkyl, $C_2$-$C_{10}$-Alkenyl, die jeweils linear oder verzweigt sein können; Aryl, Arylalkylen, bei denen die Ringsysteme jeweils substituiert sein können;

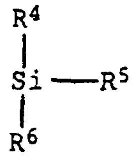

$R^2$, $R^3$ unabhängig voneinander $C_1$-$C_{10}$-Alkyl, das linear oder verzweigt sein kann;
Aryl, Arylalkylen, bei denen die Ringsysteme jeweils substituiert sein können;

$R^4$, $R^5$, $R^6$ unabhängig voneinander $C_1$-$C_{10}$-Alkyl, das linear oder verzweigt sein kann;
Aryl, Arylalkylen, bei denen die Ringsysteme jeweils substituiert sein können.

Bei den erfindungsgemäßen Diphosphinliganden der Formel I sind als Acylreste für $R^1$ verzweigte oder unverzweigte, gesättigte oder ungesättigte, gegebenenfalls mehrfach ungesättigte $C_1$-$C_{10}$-Acylketten, insbesondere Formyl, Acetyl, Propionyl, n-Butyryl, iso-Butyryl, Pivaloyl zu verstehen.

Als Alkylreste $R^1$ bis $R^6$ seien verzweigte oder unverzweigte $C_1$-$C_{10}$-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl genannt.

Als Alkenylreste $R^1$ seien verzweigte oder unverzweigte $C_2$-$C_{10}$-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Unter Aryl für $R^1$ bis $R^6$ sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z. B. Fluor, Chlor oder Brom, Cyano, Nitro, Amino, $C_1$-$C_4$-Alkylamino, $C_1$-$C_4$-Dialkylamino, Hydroxy, $C_1$-$C_4$-Alkyl, $C_1$-$C_4$-Alkoxy oder anderen Resten, insbesonders Sulfonsäure substituiert sein können. Bevorzugt sind gegebenenfalls substituiertes Phenyl, Methoxyphenyl und Naphthyl.

Als Arylalkylenreste für $R^1$ bis $R^6$ seien verzweigte oder unverzweigte Phenyl-($C_1$-$C_5$-Alkylen)- oder Naphthyl-($C_1$-$C_5$-Alkylen)-Reste wie Phenylmethylen, Phenylethylen, Phenylpropylen, Phenyl-1-methylethylen, Phenylbutylen, Phenyl-1-methylpropylen, Phenyl-2-methylpropylen, Phenyl-1,1-dimethylethylen, Phenylpentylen, Phenyl-1-methylbutylen, Phenyl-2-methylbutylen, Phenyl-3-methylbutylen, Phenyl-2,2-dimethylpropylen, Phenyl-1-ethylpropylen, Naphthylmethylen, Naphthylethylen, Naphthylpropylen, Naphthyl-1-methylethylen, Naphthylbutylen, Naphthyl-1-methylpropylen, Naphthyl-2-methylpropylen, Naphthyl-1,1-dimethylethylen, Naphthylpentylen, Naphthyl-1-methylbutylen, Naphthyl-2-methylbutylen, Naphthyl-3-methylbutylen, Naphthyl-2,2-dimethylpropylen, oder Naphthyl-1-ethylpropylen, sowie ihre isomeren oder stereoisomeren Formen genannt. Als Arylreste seien substituierte oder unsubstituierte Phenyl- oder Naphthylreste genannt.

Weiterhin sind unter Alkylenarylreste für $R^1$ $C(Ph)_3$ zu verstehen, bei dem die Phenylringe ggf. mit einem oder mehreren Resten wie Halogen z.B. Fluor, Chlor oder Brom, Cyano, Nitro, Amino, $C_1$-$C_4$-Alkylamino, $C_1$-$C_4$-Dialkylamino, Hydroxy, $C_1$-$C_4$-Alkyl, $C_1$-$C_4$-Alkoxy oder anderen Resten substituiert sein können

Besonders bevorzugt sind optisch aktive Diphosphinliganden der Formel Ia,

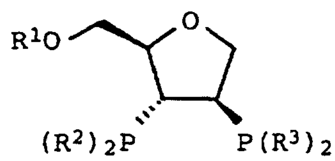

Ia

in der die Substituenten folgende Bedeutung haben:

$R^1$ $C(CH_3)_3$, $C(Ph)_3$,

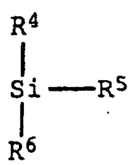

,

$R^2$, $R^3$ unabhängig voneinander Aryl, Arylalkylen, bei denen die Ringsysteme jeweils substituiert sein können,

$R^4$,$R^5$,$R^6$ unabhängig voneinander $C_1$-$C_{10}$-Alkyl, das linear oder verzweigt sein kann, Aryl, Arylalkylen, bei denen die Ringsysteme jeweils substituiert sein können.

Die Erfindung betrifft außerdem ein Verfahren zur Herstellung von optisch aktiven Diphosphinliganden der Formel I, dadurch gekennzeichnet, daß

a) ein optisch aktives, $\alpha,\beta$-ungesättigtes Lakton der allgemeinen Formel II

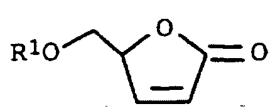

II

als Michaelakzeptor mit dem Anion von $HP(R^2)_2$ und anschließend mit $HalP(R^3)_2$ umgesetzt wird, wobei die Substituenten $R^1$ bis $R^3$ die gemäß Anspruch 1 genannte Bedeutung haben,

b) die Carbonylfunktion des Diphosphin-substituierten Lactons der allgemeinen Formel III

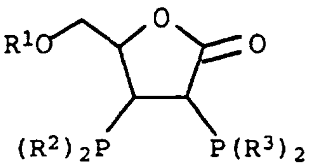

III

zur Methylengruppe reduziert wird.

Gegenstand der Erfindung sind somit auch optisch aktive Diphosphinliganden der Formel III, in der die Substituenten die gemäß Anspruch 1 genannte Bedeutung haben.

Das als "Michaelakzeptor" fungierende, optisch aktive $\alpha,\beta$-ungesättigte Lakton II läßt sich in bekannter Weise entweder aus D-Ribono-1,4-lacton gemäß Tetrahedron 43 (21), 5055 (1987) oder, wie in Tetrahedron 38 (15), 2377 (1982) beschrieben, aus Glycerinaldehyd herstellen.

Das Anion $P(R^2)_2^{\ominus}$ kann aus dem entsprechenden Phosphin $[HP(R^2)_{2]}$ in Gegenwart einer starken Base erzeugt werden. Als starke Base lassen sich u.a. Lithiumorganische Verbindungen, beispielsweise Butyllithium oder Lithiumdiisopropylamid (LDA) sowie Alkoholate in an sich bekannter Weise einsetzen. Als Gegenion (Kation) können neben Natrium, Kalium, Magnesium sowie Aluminium bevorzugt Lithium fungieren.

Die Einführung des zweiten Phosphinrestes kann durch Halogendialkylphosphine oder Halogendiarylphosphine, bevorzugt durch Halogendiarylphosphine, insbesondere durch Chlordiphenylphosphin erfolgen.

Als Lösungsmittel für diese Reaktion können alle üblichen inerten Solventien eingesetzt werden. Bevorzugte Lösungsmittel sind beispielsweise Toluol, Hexan, Cyclohexan, THF, MTB sowie Diethylether.

Die Umsetzung kann bei Temperaturen zwischen -100°C und +50°C, vorzugsweise zwischen -80°C und +30°C unter striktem Ausschluß von Sauerstoff erfolgen.

Aufgrund der hohen Hydrolyse- und Oxidationsempfindlichkeit der gebildeten Diphosphin-substituierten Laktone der Formel III, ist es vorteilhaft, diese durch Umsetzung mit $BH_3$ in die entsprechenden stabilen Boranaddukte zu überführen.

Die Reduktion der Laktone der Formel III bzw. ihrer Boranaddukte zu den entsprechenden cyclischen Äthern kann in an sich bekannter Weise (Organikum, 19. Auflage, Edition Deutscher Verlag der Wissenschaften, 1993, S. 510 - 514) durch komplexe Metallhydride wie beispielsweise $LiAlH_4$, $NaBH_4$ oder DIBAH erfolgen. Bevorzugtes Reduktionsmittel ist Diisobutylaluminiumhydrid (DIBAH) in Kombination mit Silanen z.B. $Et_3SiH$, $Ph_3SiH$ oder $PhSiH_3$ sowie mit $BF_3$ zur anschließenden Reduktion des Laktols.

Die Freisetzung der optisch aktiven Diphosphinliganden aus ihren Boranaddukten kann durch Behandlung mit basischen Reagentien, bevorzugt organische Amine, insbesondere durch Behandlung mit Diethylamin oder Triethylendiamin (1,4-Diazabicyclo[2.2.2.]octan, DABCO) erfolgen. Die Spaltung erfolgt dabei bevorzugt durch kurzzeitiges Erhitzen des Boranadduktes in Gegenwart des Amins und anschließendes mehrmaliges Abdampfen der flüchtigen Bestandteile.

Das folgende Reaktionsschema zeigt beispielhaft ein bevorzugtes Herstellverfahren für die erfindungsgemäßen Diphosphine.

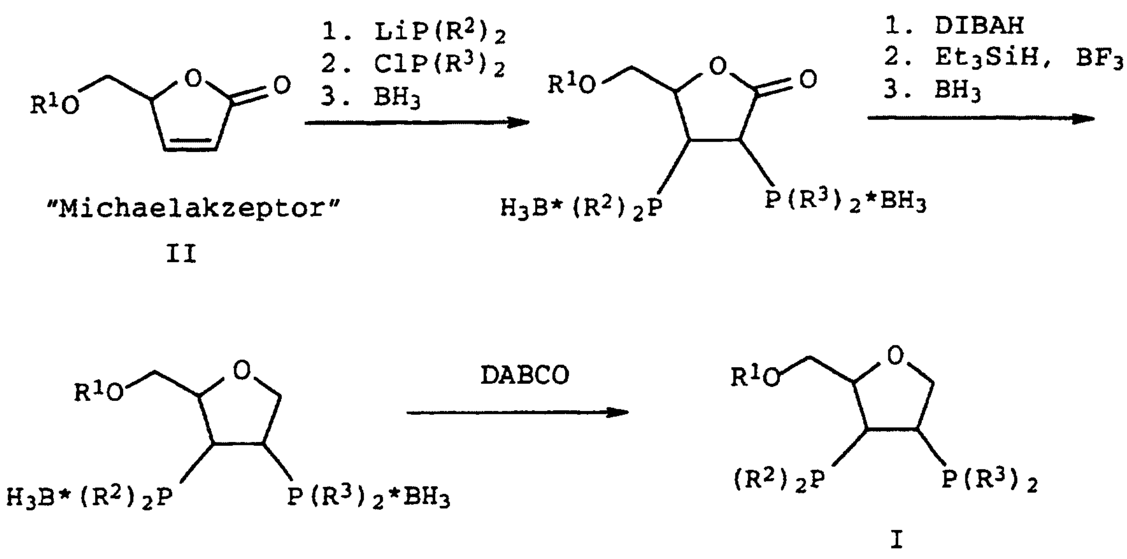


"Michaelakzeptor"
II

I

Die neuen optisch aktiven Diphosphinliganden eignen sich hervorragend zur Komplexierung von Übergangsmetallverbindungen.

Gegenstand der Erfindung sind daher auch Übergangsmetallkomplexe der allgemeinen Formel IV,

$$[M(L_n)(A)]^{p(+)}\ D^{q(-)} \qquad IV$$

in der die Variablen folgende Bedeutung haben:

M Co, Ir, Ni, Pd, Pt, Rh, Ru,

A ein optisch aktiver Diphosphinligand der Formel I, besonders bevorzugt der Formel Ia,

L ein organischer Ligand,

D das Äquivalent eines nicht koordinierten Anions,

n 1 oder 2,

p,q 0 bis 4

M ist bevorzugt ein Metall aus der Gruppe Ru, Rh und Ir. D bedeutet vorzugsweise $CF_3COO^-$, $BF_4^-$, $SbF_6^-$, $SbCl_6^-$, $PF_6^-$, $(C_6H_5)_4B^-$.

L bedeutet im Rahmen der vorliegenden Erfindung einen leicht austauschbaren Liganden wie Olefine, beispielsweise Aethylen, Propylen, Cycloocten, 1,5-Hexadien oder 1,5-Cyclooctadien.

Außerdem ist Gegenstand der Erfindung ein Verfahren zur Herstellung der oben genannten Übergangsmetallkomplexe, das dadurch gekennzeichnet ist, daß man einen optisch aktiven Diphosphinliganden der Formel I in an sich bekannter Weise mit einer Übergangsmetallverbindung $[ML_n]^{p(+)} D^{q(-)}$, in der L für einen austauschbaren Liganden der o.g. Definition steht, umsetzt.

So können beispielsweise aus diesen neuen Phosphinen in bekannter Weise [z.B. Uson, Inorganic Chim. Acta 73, 275 (1983), EP-A-0 158 875, EP-A-0 437 690] durch Umsetzung mit Übergangsmetallkomplexen, die labile Liganden enthalten, insbesondere mit $[RuCl_2(COD)]_n$, $Rh(COD)_2BF_4$, $Rh(COD)_2ClO_4$, $[Ir(COD)Cl]_2$, p-Cymol-Rutheniumchlorid-dimer katalytisch aktive Komplexe synthetisiert werden.

Die Übergangsmetallkomplexe der allgemeinen Formel IV eignen sich als Katalysatoren für asymmetrische Reaktionen, insbesondere für die asymmetrische Hydrierung von Verbindungen mit C-C, C-N und C-O Mehrfachbindungen, besonders sei hier genannt die Hydrierung von β-Ketoestern, Allylalkoholen, Enamiden und Dehydroaminosäuren. Ebenso eignen sich die Übergangsmetallkomplexe der allgemeinen Formel IV für die asymmetrische Isomerisierung von Allylaminen zu Enaminen und für asymmetrische Hydroformylierungsreaktionen.

In den nachfolgenden Beispielen wird die Herstellung der erfindungsgemäßen optisch aktiven Diphosphinliganden und deren Übergangsmetallkomplexe sowie die Anwendung der Metallkomplexe für die enantioselektive Synthese näher erläutert.

Beispiel 1: Herstellung des Diphenylphosphin-Boranadduktes der Formel

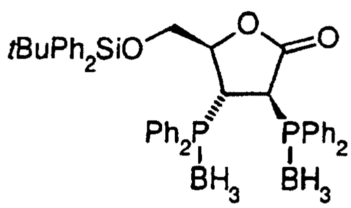

3.27 g (17.6 mmol) Diphenylphosphin wurden in 50 ml THF gelöst und bei -78 °C durch 19.1 mmol n-Butyllithium deprotoniert. Eine Lösung von 5.543 g (15.7 mmol) "Michaelakzeptor" der Formel II ($R^1$ = $tBuPh_2Si$) in 50 ml THF wurde auf -78 °C gekühlt und mittels Doppelnadel zur Phosphin-Lösung getropft. Nach einer halben Stunde gab man 4.20 g (19.1 mmol) Chlordiphenylphosphin, gelöst in 50 ml THF, zu. Man ließ die Reaktionslösung über Nacht auf Raumtemperatur auftauen und kühlte vor der Zugabe von 45 ml 1M-Boran-THF-Lösung wieder auf -78 °C. Nach einer Stunde wurde das Lösungsmittel im Feinvakuum abgedampft, der Rückstand in Essigsäureethylester aufgenommen und die Lösung mit Wasser gewaschen. Das Produkt erhielt man durch Flashchromatographie über Kieselgel in einer Ausbeute von 9.45 g (81 %) in Form eines amorphen farblosen Feststoffes.
Schmelzpunkt: 133 - 134 °C.
Optische Drehung:

$$[\alpha]^{20}_{D} = -57.1, [\alpha]^{20}_{578} = -59.9, [\alpha]^{20}_{546} = -69.1,$$

$$[\alpha]^{20}_{436} = -127.4, [\alpha]^{20}_{365} = -222.5; (c = 1.14, CHCl_3).$$

$^1H$ NMR ($CDCl_3$, 300.13 MHz): d (ppm) = 0.3 - 1.6 (bs, 6 H, $BH_3$), 0.97 (s, 9 H, $C(\underline{CH_3})_3$), 3.11 (dd, $J_{6,6'}$ = 10.9 Hz, $J_{5,6}$ = 7.0 Hz, 1 H, 6-H), 3.30 (dd, $J_{6,6'}$ = 10.7 Hz, $J_{5,6'}$ = 6.5 Hz, 1 H, 6'-H), 3.94 (ddd, $^2J_{3,P}$ = 20.2 Hz, $^3J_{3,P}$ = 10.5 Hz, $J_{3,4}$ = 3.4 Hz, 1 H, 3-H), 4.15 (m, 1 H, 4-H), 4.64 (m, 1 H, 5-H), 7.22 - 7.88 (sh, 30 H, Ar-H).
$^{13}C$ NMR ($CDCl_3$, 75.47 MHz): d (ppm) = 19.2 (s, $\underline{C}(CH_3)_3$), 26.9 (q, $CH_3$), 34.4 (d, $^1J_{C,P}$ = 38.4 Hz, C-4), 40.8 (d, $^1J_{C,P}$ = 26.0 Hz, C-3), 65.3 (t, $\underline{C}H_2OSi$), 79.1 (d, C-5), 127.7, 127.8, 128.7, 128.8, 128.9, 129.1, 129.1, 129.2, 129.8, 129.9, 131.6, 132.1, 132.3, 132.4, 132.5, 132.9, 133.1, 133.3, 133.5, 133.6, 134.1, 134.2, 135.4 (Ar), 171.0 (s, C=O).
$^{31}P$ NMR ($CDCl_3$, 81.015 MHz): d (ppm) = 23.9 (m, $P-BH_3$)

Beispiel 2: Reduktion des Lactons zum Ether der Formel

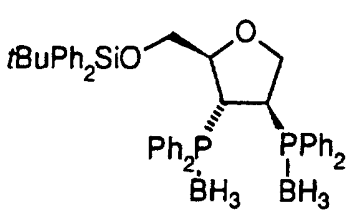

Man löste 2.1 g (2.74 mmol) des nach Beispiel 1 erhaltenen Diphosphins in 20 ml Toluol, kühlte auf -78 °C und tropfte 2.9 ml 1M-DIBAL-H-Lösung zu. Nach wäßriger Aufarbeitung wurde der Rückstand in 20 ml Dichlormethan

gelöst. Zu der auf -78 °C gekühlten Lösung gab man 666 mg (5.73 mmol) Triethylsilan und 412.3 mg (2.91 mmol) Bortrifluorid-Diethyletherat . Man ließ über Nacht auf Raumtemperatur auftauen und gab zur vollständigen Umsetzung noch drei Tropfen Bortrifluorid-Diethyletherat zu. Nach der Zugabe von 6 ml 1M-$BH_3$-THF-Lösung wurde ohne Schutzgas aufgearbeitet. Man erhielt 1.3743 g (68 %) als amorphes Pulver.
Optische Drehung:

$$[\alpha]^{20}_{D} = -25.0,\ [\alpha]^{20}_{578} = -26.2,\ [\alpha]^{20}_{546} = -30.2,$$

$$[\alpha]^{20}_{436} = -54.4,\ [\alpha]^{20}_{365} = -91.6,\ (c = 1.41,\ CHCl_3).$$

$^1H$ NMR ($CDCl_3$, 300.13 MHz): d (ppm) = 0.3 - 1.6 (bs, 6 H, $BH_3$), 0.99 (s, 9 H, C(C$\underline{H}_3$)$_3$), 3.19 (dd, $J_{6,6'}$ = 10.8 Hz, $J_{2,6'}$ = 3.8 Hz, 1 H, 6-H), 3.54 (m, 1 H, 6'-H), 3.55 (m, 1 H, 3-H), 3.77 (ddd, $J_{5,P}$ = 16.3 Hz, $J_{5,5'}$ = 8.4 Hz, $J_{5,4}$ = 5.1 Hz, 1 H, 5-H), 3.87 (m, 1 H, 4-H), 3.93 (ddd, $J_{5',4'}$ = 15.7 Hz, $J_{5',P}$ = 10.6 Hz, $J_{5',5}$ = 7.8 Hz, 1 H, 5'-H), 4.31 (m, $J_{2,P}$ = 14.7 Hz, 1 H, 2-H), 7.22 - 7.88 (sh, 30 H, Ar-H).
$^{13}C$ NMR ($CDCl_3$, 75.47 MHz): d (ppm) = 19.0 (s, $\underline{C}(CH_3)_3$), 26.6 (q, $CH_3$), 35.8 (d, $J_{P,C}$ = 40.1 Hz, C-3), 35.9 (d, $J_{P,C}$ = 40.0 Hz, C-4), 64.4 (t, $J_{P,C}$ = 5.4 Hz, $\underline{C}H_2OSi$), 70.3 (t, $J_{P,C}$ = $J_{P',C}$ = 4.2 Hz, C-5), 83.1 (d, $J_{P,C}$ = $J_{P',C}$ = 4.6 Hz, C-2), 127.3 - 135.4 (Ar-C).
$^{31}P$ NMR ($CDCl_3$, 81.015 MHz): d (ppm) = 19.6 (m, P-$BH_3$).

Beispiel 3: Freisetzung der Phosphine aus ihren Boranaddukten

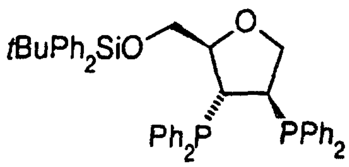

1 mmol Boranaddukt aus Beispiel 2 wurde mit 0.5 ml Diethylamin versetzt und 30 min am Rückfluss gerührt. Anschließend wurden die flüchtigen Bestandteile zweimal im Hochvakuum entfernt. Die freien Phosphine wurden als luftempfindliche weiße Pulver erhalten.

Beispiel 4: Herstellung des Rhodiumkomplexes und asymmetrische Hydrierung von α-Acetamidozimtsäure

110 µmol des optisch aktiven Diphenylphosphin Liganden nach Beispiel 3 wurden in 5 ml THF gelöst, mit 100 µmol $Rh(COD)_2BF_4$ versetzt und 20 min bei RT gerührt. Zu der klaren gelben Lösung wurden 100 mmol α-Acetamidozimtsäure und 250 ml THF gegeben. Diese Lösung wurde bei 30°C und 20 bar Wasserstoff hydriert. Die Reaktion war nach 20 min vollständig. N-Acetyl-(S)-Phenylalanin wurde nach Aufarbeitung in 97 % Ausbeute und einer optischen Reinheit von 99.6 % ee isoliert.

Beispiel 5: Herstellung von N-Acetyl-(S)-Cyclohexylalanin

110 µmol des optisch aktiven Diphenylphosphin Liganden nach Beispiel 3 wurden in 5 ml THF gelöst, mit 100 µmol $Rh(COD)_2BF_4$ versetzt und 20 min bei RT gerührt. Zu der klaren gelben Lösung wurden 100 mmol α-Acetamidozimtsäure und 250 ml THF gegeben. Diese Lösung wurde bei 30°C und 20 bar Wasserstoff hydriert. Die Reaktion war nach 20 min vollständig. Anschließend wurde der Autoklav geöffnet und zur Oxidation des Phosphinliganden 5 min Luft eingeleitet. Nach Zugabe von 0.5 g Rutheniumoxidhydrat wurde innerhalb von 3 h bei 160°C und 100 bar Wasserstoff eine Kernhydrierung durchgeführt. N-Acetyl-(S)-Cyclohexylalanin wurde in > 95%iger Ausbeute mit einem ee-Wert von 99.6 % isoliert.

**Patentansprüche**

**1.** Optisch aktive Diphosphinliganden der Formel I

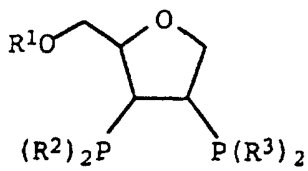

I

in der die Substituenten folgende Bedeutung haben:

| | |
|---|---|
| $R^1$ | Wasserstoff,<br>$C_1$-$C_{10}$-Acyl, $C_1$-$C_{10}$-Alkyl, $C_2$-$C_{10}$-Alkenyl, die jeweils linear oder verzweigt sein können,<br>Aryl, Arylalkylen, bei denen die Ringsysteme jeweils substituiert sein können, |

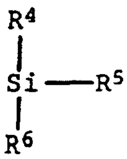

| | |
|---|---|
| $R^2$, $R^3$ | unabhängig voneinander $C_1$-$C_{10}$-Alkyl, das linear oder verzweigt sein kann,<br>Aryl, Arylalkylen, bei denen die Ringsysteme jeweils substituiert sein können, |
| $R^{4'}$ $R^5$, $R^6$ | unabhängig voneinander $C_1$-$C_{10}$-Alkyl, das linear oder verzweigt sein kann,<br>Aryl, Arylalkylen, bei denen die Ringsysteme jeweils substituiert sein können, |

sowie deren Boran-Addukte.

**2.** Optisch aktive Diphosphinliganden der Formel Ia,

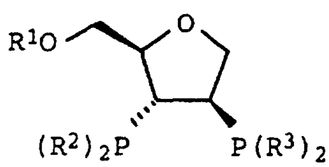

Ia

in der die Substituenten die gemäß Anspruch 1 genannte Bedeutung haben, sowie deren Boran-Addukte.

**3.** Verfahren zur Herstellung optisch aktiver Diphosphinliganden der Formel I, **dadurch gekennzeichnet, daß**

a) ein optisch aktives, $\alpha,\beta$-ungesättigtes Lakton der allgemeinen Formel II

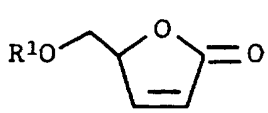

II

als Michaelakzeptor mit dem Anion von $HP(R^2)_2$ und anschließend mit $HalP(R^3)_2$ umgesetzt wird, wobei die Substituenten $R^1$ bis $R^3$ die gemäß Anspruch 1 genannte Bedeutung haben, und

0 b) die Carbonylfunktion des Diphosphin-substituierten Lactons der allgemeinen Formel III

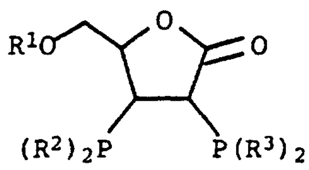

III

zur Methylengruppe reduziert wird.

**4.** Optisch aktive Diphosphinliganden der Formel III, in der die Substituenten die gemäß Anspruch 1 genannte Bedeutung haben.

**5.** Verwendung der optisch aktiven Diphosphinliganden der Formel I gemäß Anspruch 1 zur Komplexierung von Übergangsmetallverbindungen.

**6.** Übergangsmetallkomplexe der Formel IV

$$[M(L_n)\ (A)]^{p(+)}\ D^{q(-)} \qquad IV$$

in der die Variablen folgende Bedeutung haben:

M Co, Ir, Ni, Pd, Pt, Rh, Ru,

A ein optisch aktiver Diphosphinligand der Formel I gemäß Anspruch 1,

L ein organischer Ligand,

D das Äquivalent eines nicht koordinierten Anions,

n 1 oder 2,

p,q 0 bis 4

**7.** Verfahren zur Herstellung der Übergangsmetallkomplexe gemäß Anspruch 6, **dadurch gekennzeichnet, daß** man einen optisch aktiven Diphosphinliganden der Formel I gemäß Anspruch 1 in an sich bekannter Weise mit einer Übergangsmetallverbindung $[ML_n]^{p(+)}\ D^{q(-)}$, in der die Variablen die gemäß Anspruch 6 genannte Bedeutung haben, umsetzt.

**8.** Verwendung der Metallkomplexe der Formel IV gemäß Anspruch 6 als Katalysatoren für die asymmmetrische Hydrierung von β-ketoestern, Allylalkoholen, Enamiden und Dehydroaminosäuren

**9.** Verwendung der Metallkomplexe der Formel IV gemäß Anspruch 6 als Katalysatoren für die asymmmetrische Isomerisierug von Allylaminen zu Enaminen.

## Claims

**1.** An optically active diphosphine ligand of the formula I

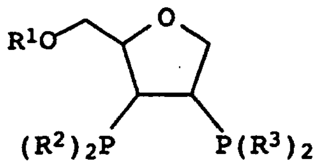

I

where:

$R^1$ is hydrogen, $C_1$-$C_{10}$-acyl, $C_1$-$C_{10}$-alkyl, $C_2$-$C_{10}$-alkenyl, each of which may be linear or branched, aryl, arylalkyl, in which the ring systems may be substituted in each case,

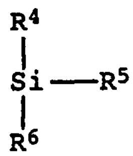

$R^2$, $R^3$ independently of one another are $C_1$-$C_{10}$-alkyl, which may be linear or branched, aryl, arylalkyl, in which the ring systems may be substituted in each case,

$R^4$,$R^5$, $R^6$ independently of one another are $C_1$-$C_{10}$-alkyl, which may be linear or branched, aryl, arylalkyl, in which the ring systems may be substituted in each case,

or its borane adduct.

**2.** An optically active diphosphine ligand of the formula Ia,

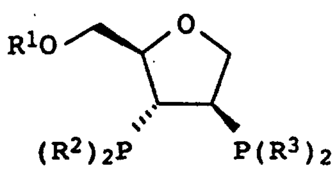

Ia

where the substituents have the meanings stated in claim 1, or its borane adduct.

**3.** A process for preparing optically active diphosphine ligands of the formula I, which comprises

a) reacting an optically active $\alpha,\beta$-unsaturated lactone of the formula II

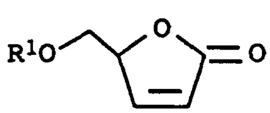

II

as Michael acceptor with the anion of $HP(R^2)_2$ and then with $HalP(R^3)_2$, where $R^1$ to $R^3$ have the meanings stated in claim 1, and

b) reducing the carbonyl functionality of the diphosphine-substituted lactone of the formula III

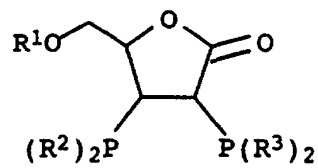

III

to the methylene group.

**4.** An optically active diphosphine ligand of the formula III, where the substituents have the meanings stated in claim 1.

**5.** The use of an optically active diphosphine ligand of the formula I as claimed in claim 1 for complexing transition metal compounds.

**6.** A transition metal complex of the formula IV

$$[M(L_n)\,(A)]^{p(+)}\,D^{q(-)} \qquad IV$$

where:

M is Co, Ir, Ni, Pd, Pt, Rh, Ru,

A is an optically active diphosphine ligand of the formula I as claimed in claim 1,

L is an organic ligand,

D is the equivalent of a non-coordinated anion,

n is 1 or 2,

p, q are 0 to 4

**7.** A process for preparing a transition metal complex as claimed in claim 6, which comprises reacting an optically active diphosphine ligand of the formula I as claimed in claim 1 in a manner known per se with a transition metal compound $[ML_n]^{p(+)}\,D^{q(-)}$, where the variables have the meanings stated in claim 6.

**8.** The use of a metal complex of the formula IV as claimed in claim 6 as catalyst for the asymmetric hydrogenation of β-keto esters, allyl alcohols, enamides and dehydro amino acids.

**9.** The use of a metal complex of the formula IV as claimed in claim 6 as catalyst for the asymmetric isomerization of allylamines to enamines.

**Revendications**

**1.** Ligands de type diphosphine, possédant l'activité optique et répondant à la formule I

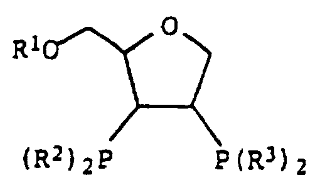

I

dans laquelle les symboles ont les significations suivantes :

$R^1$. l'hydrogène,
un groupe acyle en C1-C10, alkyle en C1-C10, alcényle en C2-C10, chacun d'eux linéaire ou ramifié,
un groupe aryle, arylalkylène, dont les systèmes cycliques peuvent être substitués,

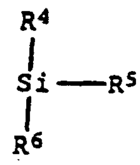

$R^2$, $R^3$. chacun, indépendamment l'un de l'autre, un groupe alkyle en C1-C10 à chaîne droite ou ramifiée,
un groupe aryle, arylalkylène, dont les systèmes cycliques peuvent être substitués $R^4$, $R^5$, $R^6$ : chacun, indépendamment les uns des autres, un groupe alkyle en C1-C10 à chaîne droite ou ramifiée,
un groupe aryle, arylalkylène, dans lesquels les systèmes cycliques peuvent être substitués,

et leurs adducts avec un borane.

2. Ligands du type diphosphine, possédant l'activité optique, et répondant à la formule Ia

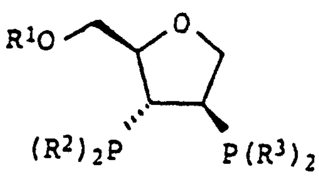

Ia

dans laquelle les symboles ont les significations indiquées dans la revendication 1, et leurs adducts avec un borane.

3. Procédé pour la préparation de ligands du type diphosphine, possédant l'activité optique et répondant à la formule I, **caractérisé en ce que**

a) on fait réagir une lactone alpha,bêta-insaturée, possédant l'activité optique, de formule générale II

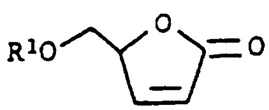

II

en tant qu'accepteur de Michael, avec l'anion de $HP(R^2)_2$ puis avec $HalP(R^3)_2$, les symboles $R^1$ à $R^3$ ayant les significations indiquées dans la revendication 1, et
b) on réduit en groupe méthylène la fonction carbonyle de la lactone à substituant diphosphine de formule générale III

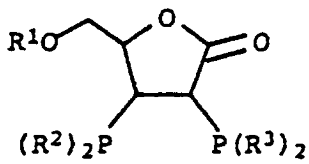

III

4. Ligands du type diphosphine, possédant l'activité optique et répondant à la formule III dans laquelle les symboles ont les significations indiquées dans la revendication 1.

5. Utilisation des ligands du type diphosphine, possédant l'activité optique et répondant à la formule I de la revendication 1, pour la complexation de dérivés de métaux de transition.

6. Complexes de métaux de transition répondant à la formule IV

$$[M(L_n)\ (A)]^{p(+)}\ D^{q(-)} \qquad \text{IV}$$

dans laquelle les symboles ont les significations suivantes

M représente Co, Ir, Ni, Pd, Pt, Rh, Ru,
A représente un ligand du type diphosphine possédant l'activité optique et répondant à la formule I de la revendication 1,
L représente un ligand organique,
D représente l'équivalent d'un anion non coordinant,
N est égal à 1 ou 2,
p et q sont des nombres allant de 0 à 4.

7. Procédé pour la préparation des complexes de métaux de transition selon la revendication 6, **caractérisé par le fait que** l'on fait réagir des ligands du type diphosphine possédant l'activité optique et répondant à la formule I de la revendication 1, de manière connue en soi, avec un dérivé de métal de transition $[ML_n]^{p(+)}\ D^{q(-)}$ pour lequel les symboles ont les significations indiquées dans la revendication 6.

8. Utilisation des complexes métalliques de formule IV de la revendication 6 en tant que catalyseurs pour l'hydrogénation asymétrique de bêta-cétoesters, d'alcools allyliques, d'énamides et de déshydroaminoacides.

9. Utilisation des complexes métalliques de formule IV de la revendication 6 en tant que catalyseurs pour l'isomérisation asymétrique d'allylamines en énamines.